# FASCICULE DE BREVET EUROPEEN

(11) **EP 0 770 374 B1**
(45) Date de publication et mention de la délivrance du brevet: **29.07.1998**
(21) Numéro de dépôt: 96402171.1
(22) Date de dépôt: 11.10.1996
(51) Int. Cl.: A61K 7/09

(54) **Composition pour la déformation permanente des fibres kératiniques, contenant un antagoniste de substance P ou un antagoniste de CGRP**
Zubereitung zur dauerhaften Deformation von Keratinenfasern, die eine Substanz P- oder CGRP-Antagonisten enthalten
Compsition for the permanent deformation of keratinin fibres containing a substance P or CGRP antagonist

(30) Priorité: 26.10.1995 FR 9512654
(43) Date de publication de la demande: 02.05.1997
(60) Demande divisionnaire: 97115409.1
(73) Titulaire: L'OREAL, 75008 Paris (FR)
(72) Inventeur: de Lacharriere, Olivier, 75015 Paris (FR); Loussouarn, Geneviève, 92110 Clichy (FR); Breton, Lionel, 78000 Versailles (FR)
(74) Mandataire: Lhoste, Catherine

(56) Documents cités:
- EP-A- 0 235 783
- EP-A- 0 237 870
- EP-A- 0 461 526
- EP-A- 0 624 572
- EP-A- 0 625 350
- DE-A- 3 535 351
- DE-A- 3 610 394
- DE-A- 4 336 838
- FR-A- 2 707 486
- FR-A- 2 718 351
- NEUROSCIENCE, vol. 48, no. 4, 1992, pages 963-968, XP000575623 T.L. BUCKLEY ET AL:
- BRITISH JOURNAL OF PHARMACOLOGY, vol. 110, no. 2, Octobre 1993, pages 772-776, XP000563605 K.JANE ESCOTT ET AL:
- BRITISH JOURNAL OF PHARMACOLOGY, vol. 104, no. 3, 1991, pages 738-742, XP002007864 S.R. HUGHES ET AL:

## Description

La présente invention se rapporte à une composition notamment cosmétique, et plus particulièrement pour la déformation permanente des fibres kératiniques, contenant au moins un antagoniste de substance P et/ou un antagoniste de CGRP, notamment pour diminuer voire éliminer les effets irritants des agents contenus dans une telle composition.

On sait que la technique la plus usuelle pour obtenir une déformation permanente des cheveux, appelée ci-après 〈〈 permanente 〉〉, consiste, dans un premier temps, à réaliser l'ouverture des liaisons disulfures -S-S- de la kératine (cystine) à l'aide d'une composition contenant un agent réducteur (étape de réduction) puis, après avoir rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action de l'eau ou aux lavages avec un shampooing, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites, des alkyl-phosphines ou de préférence des thiols. Parmi ces derniers, ceux couramment utilisés sont la cystéïne et ses dérivés, la cystéamine et ses dérivés, l'acide thiolactique et ses esters, l'acide thioglycolique ainsi que ses esters, notamment le monothioglycolate de glycérol.

Concernant les compositions oxydantes nécessaires à la mise en oeuvre de l'étape de fixation, on fait le plus souvent appel, dans la pratique, à des compositions à base d'eau oxygénée ou de bromate alcalin.

Certains agents réducteurs et oxydants, ainsi que certains adjuvants utilisés pour les permanentes peuvent entraîner des irritations et/ou des sensations d'inconfort (par exemple fourmillements, échauffements, démangeaisons), et cet effet irritant est ressenti en particulier chez certaines personnes présentant un cuir chevelu sensible.

En effet, certains sujets présentent une réactivité du cuir chevelu plus importante que d'autres. En particulier, ils répondent à certains produits tels que les colorants capillaires, les permanentes, les tensioactifs. Les individus, outre les signes cliniques mentionnés ci-dessus, ont la particularité d'avoir une tendance à répondre plus fortement à certains tests de provocation qui visent à reproduire ces signes cliniques. Parmi ces tests, la demanderesse a mis au point le test à la capsaïcine et celui à la monoéthanolamine.

Le test à la capsaïcine est décrit dans la demande de brevet français n° 94-05537 déposée par la demanderesse.

Le test à la monoéthanolamine consiste à préparer une solution aqueuse contenant 10 % de monoéthanolamine, à verser sur un coton 3 ml de cette solution, à appliquer ce coton 10 fois sur les zones testées du cuir chevelu et à évaluer les signes cliniques qui apparaissent à 30 secondes, 2 minutes, 5 minutes, 10 minutes et 15 minutes après application. Ces signes sont essentiellement des signes subjectifs (picotements, fourmillements, démangeaisons, échauffements), éventuellement associés à un érythème.

Or, la demanderesse a découvert que l'utilisation d'antagonistes de substance P et/ou d'antagonistes de CGRP permettait d'obtenir un effet préventif et/ou curatif de l'irritation dûe aux composés utilisés dans les compositions de permanente.

La substance P est un élément chimique polypeptidique élaboré et libéré par les terminaisons nerveuses. Elle fait partie de la famille des tachykinines. La substance P intervient notamment dans la transmission de la douleur, dans des maladies du système nerveux central, telles que l'anxiété et la schizophrénie, dans des maladies respiratoires et inflammatoires, dans des maladies gastrointestinales, dans des maladies rhumatismales et dans certaines maladies dermatologiques telles que l'eczéma.

Le CGRP (peptide dérivé du gène de la calcitonine : Calcitonin Gene Related Peptide en langue anglosaxonne) est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

Personne jusqu'à ce jour n'avait envisagé d'utiliser des antagonistes de substance P ou de CGRP en association avec des composés utilisés dans les permanentes et notamment les matières réductrices et/ou oxydantes, en vue d'éliminer l'effet irritant et/ou d'inconfort provoqués par ces composés.

En outre, il est connu du document FR-M-5394 un médicament contenant des crésols et du chlorure de strontium pour augmenter les propriétés antibactériennes et antimicrobiennes de ces crésols. Toutefois, ce document ne décrit ni ne suggère que le chlorure de strontium puisse diminuer voire éliminer, à lui seul, les symptômes tels que les irritations, douleurs, démangeaisons, picotements, causés par une quelconque maladie, ni diminuer voire supprimer ces mêmes symptômes causés par un produit irritant.

En outre, le document EP-A-716850 opposable au titre de l'article 54 (3) CBE décrit une composition contenant un dérivé d'éthylènediamine, antagoniste de substance P et un thiol. Par ailleurs, le document EP-A-723774 opposable également au titre de l'article 54 (3) CBE décrit une composition contenant un antagoniste de CGRP et un thiol.

Aussi, la présente invention a pour objet une composition notamment cosmétique contenant dans un milieu physiologiquement acceptable au moins un antagoniste de substance P et au moins un actif à effet secondaire irritant, caractérisée en ce que l'actif à effet secondaire irritant est un agent réducteur et/ou un agent oxydant à l'exception des peroxydes et des crésols, à condition que l'actif soit différent d'un thiol quand l'antagoniste de substance P est un dérivé d'éthylène diamine.

La présente invention a aussi pour objet une composition notamment cosmétique contenant dans un milieu physiologiquement acceptable au moins un antagoniste de CGRP et au moins un actif à effet secondaire irritant, caractérisée en ce que l'actif à effet secondaire irritant est un agent réducteur et/ou un agent oxydant à l'exception des peroxydes, des crésols et des thiols.

La composition selon l'invention est notamment une composition pour la déformation permanente des fibres kératiniques, et en particulier une composition pour la déformation permanente des cheveux humains.

Aussi, la présente invention a encore pour objet une composition de déformation permanente des fibres kératiniques contenant, dans un milieu approprié pour la déformation permanente, au moins un agent réducteur et/ou un agent oxydant à l'exception des crésols, caractérisée en ce que, l'agent réducteur et/ou oxydant ayant un effet secondaire irritant, elle contient en outre au moins un antagoniste choisi parmi les antagonistes de substance P et les antagonistes de CGRP.

L'invention a encore pour objet l'utilisation de la monoéthanolamine pour préparer une composition destinée à déterminer les cuirs chevelus sensibles.

L'antagoniste de substance P ou de CGRP peut être appliqué sur les fibres kératiniques avant et/ou pendant et/ou après les étapes de réduction et/ou d'oxydation.

Aussi, l'invention a encore pour objet un procédé de traitement des fibres kératiniques, en particulier des cheveux, en vue d'obtenir une déformation permanente de ces fibres, caractérisé par le fait qu'il comprend les étapes suivantes :
i) application sur les fibres kératiniques d'une composition réductrice, les moyens nécessaires à la mise sous tension mécanique des fibres kératiniques étant mis en oeuvre avant, pendant ou après cette application de la composition réductrice,
ii) après action de la composition réductrice, rinçage des fibres kératiniques,
iii) application sur les fibres kératiniques d'une composition oxydante,
iv) séparation des moyens de mise sous tension avant ou après l'étape iii,
v) rinçage éventuel des fibres kératiniques,
vi) application sur les fibres kératiniques d'une composition contenant au moins un antagoniste choisi parmi les antagonistes de substance P et les antagonistes de CGRP, au cours de l'une au moins des étapes i à v et/ou après l'une au moins de ces étapes i à v.

Selon un mode particulier de réalisation de l'invention, les différentes compositions sont conditionnées séparément sous forme d'un kit, selon un agencement bien connu de l'homme du métier.

Aussi, l'invention a encore pour objet un kit pour le traitement des fibres kératiniques, en particulier des cheveux, en vue d'obtenir une déformation permanente de ces fibres, comprenant une première composition contenant un agent réducteur et une seconde composition contenant un agent oxydant, les deux compositions, destinées à être appliquées l'une après l'autre sur les fibres kératiniques, étant conditionnées séparément, l'une au moins de ces compositions contenant un antagoniste choisi parmi les antagonistes de substance P et les antagonistes de CGRP.

L'invention a également pour objet un kit pour le traitement des fibres kératiniques, en particulier des cheveux, en vue d'obtenir une déformation permanente de ces fibres, comprenant une première composition contenant un agent réducteur, une seconde composition contenant un agent oxydant, une troisième composition contenant un antagoniste choisi parmi les antagonistes de substance P et les antagonistes de CGRP, les trois compositions, destinées à être appliquées les unes après les autres sur les fibres kératiniques, étant conditionnées séparément.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action. Il peut être choisi parmi les composés connus comme antagonistes de substance P, notamment les peptides, les dérivés non peptidiques, et plus précisément ceux comportant un hétérocycle azoté, soufré ou oxygéné, ou les composés azotés comportant un atome d'azote lié directement ou indirectement à un cycle benzénique. Il peut être choisi également parmi les sels de métaux monovalents, divalents ou trivalents, et parmi des extraits d'origine végétale et/ou bactérienne.

Ainsi, on peut utiliser dans l'invention par exemple comme peptide antagoniste de substance P le sendide et le spantide II. On peut également utiliser les peptides décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés hétérocycliques, notamment azotés, soufrés ou oxygénés, ou des composés comprenant un atome d'azote lié directement ou indirectement à un ou plusieurs cycles benzéniques.

Comme composé hétérocyclique, on peut utiliser dans l'invention ceux comportant un hétérocycle azoté, décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle ou un dérivé d'isoindole.

Comme autres composés hétérocycliques, on peut citer les composés hétérocycliques oxygénés ou soufrés tels que les dérivés du furanne, les dérivés du benzofuranne, les dérivés du thiophène et les dérivés du benzothiophène, comportant éventuellement des substituants azotés, tels que les composés hétérocycliques décrits dans les documents US-A-4931459, US-A-4910317 et EP-A-299457, et plus spécialement les alcoxy- et/ou aryloxy- tétrazolyl-benzofuranne-carboxamides ou les alcoxy- et/ou aryloxy- tétrazolyl-benzothiophène-carboxamides.

Comme composés comportant un atome d'azote lié directement ou indirectement à un noyau benzénique, on peut citer ceux décrits dans les documents suivants : EP-A-522808, WO-A-93/01165 et WO-A-93/10073. On peut citer notamment les dérivés d'éthylène diamine, tels que la N,N'-bis-di-(3,5-dimethylbenzyle)- éthylène diamine, la N,N'-bis-di-(3,5-dimethoxybenzyle)-éthylène diamine ; ces composés sont décrits comme intermédiaires de synthèse dans le document WO-A-93/11338 déposé au nom de la demanderesse.

Les sels de métaux monovalents, divalents ou trivalents, utilisables dans l'invention comme antagonistes de substance P, peuvent être des sels de cobalt ; des sels des éléments de la colonne II A de la classification périodique, notamment des sels de béryllium, de magnésium ou de métal alcalino-terreux, en particulier de strontium, de calcium et de baryum ; des sels de lanthanides, notamment de lanthane et de gadolinium ; des sels d'yttrium ; des sels de zinc ; des sels de manganèse ; des sels de cuivre ; des sels de rubidium ; des sels de lithium.

Ces sels peuvent être par exemple des chlorures, des carbonates, des bicarbonates, des borates, des nitrates, des acétates, des hydroxydes, des sulfates, des persulfates, des glycérophosphates, ainsi que des sels d'a-hydroxyacides ou des sels d'acides de fruits (citrate, tartrate, lactate, malate), ou encore des sels d'acides aminés (aspartate, arginate, glucocholate, fumarate) ou des sels d'acides gras (palmitate, oléate, caséinate, béhénate).

De façon avantageuse, le sel est un sel de strontium, et notamment le chlorure de strontium ou le nitrate de strontium.

Les extraits d'origine bactérienne utilisables dans l'invention peuvent être des extraits d'au moins une bactérie filamenteuse non photosynthétique.

Comme extrait d'origine végétale utilisable dans l'invention, on peut citer notamment ceux provenant d'*Iris germanica,* d'*Iris florentina,* d'*Iris pallida,* de *Crocus versicolor,* de *Romulea bulbucodium* ou encore de *Gladiolus communis.* Plus particulièrement selon l'invention, on utilise un extrait végétal issu d'une iridacée et préférentiellement du matériel végétal d'*Iris pallida.* Toute méthode d'extraction connue de l'homme du métier peut être utilisée pour préparer l'extrait contenu dans la composition selon l'invention. On peut, en particulier, citer les extraits alcooliques, notamment éthanoliques, ou encore hydroalcooliques. On peut également utiliser un extrait préparé par la méthode décrite dans la demande de brevet français n° 95-02379 déposée par la demanderesse.

Pour qu'une substance soit reconnue comme un antagoniste de CGRP, elle doit répondre notamment aux caractéristiques suivantes :
- avoir une affinité pour les récepteurs au CGRP et/ou
- avoir une activité pharmacologique antagoniste du CGRP, c'est-à-dire induire une réponse pharmacologique cohérente notamment dans l'un des tests suivants :
   - la substance antagoniste doit diminuer la vasodilatation induite par la capsaïcine et/ou
   - la substance antagoniste doit provoquer une inhibition de la libération de CGRP par les fibres nerveuses sensitives et/ou
   - la substance antagoniste doit provoquer une inhibition de la contraction du muscle lisse du canal déférent induite par le CGRP.

On peut utiliser dans l'invention par exemple comme antagoniste de CGRP, le CGRP 8-37 (séquence des acides aminés 8 à 37 de la partie terminale du CGRP), un anticorps anti-CGRP.

Dans les compositions selon l'invention, l'antagoniste de substance P ou de CGRP est utilisé de préférence en une quantité allant de 0,000001 à 30 % en poids par rapport au poids total de la composition, et en particulier en une quantité allant de 0,0001 à 10 % en poids par rapport au poids total de la composition.

L'antagoniste de substance P ou de CGRP joue essentiellement un rôle sur les agents réducteurs et/ou oxydants ayant un effet irritant, les agents non irritants étant par ailleurs présents dans la composition selon l'invention pour permettre d'atteindre la déformation souhaitée.

Dans la première étape (i) du procédé de traitement selon l'invention, on applique sur les fibres kératiniques une composition réductrice contenant au moins un agent actif approprié pour la réduction des liaisons disulfures de la kératine. Cette application peut être réalisée avant, pendant ou après l'habituelle étape de mise sous tension des fibres kératiniques. L'antagoniste de substance P ou de CGRP peut être présent dans la composition réductrice ou dans une composition appliquée sur les fibres kératiniques avant ou après l'étape de réduction.

L'habituelle étape de mise sous tension des fibres kératiniques, notamment des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.

Parmi les agents actifs appropriés pour la réduction des liaisons disulfures de la kératine, on peut citer les sulfites, les bisulfites, les alkyl-phosphines ou, de préférence, les thiols. Parmi ces derniers, ceux préférentiellement utilisés sont choisis parmi l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les esters de cystéine, tels que le cystéïnate de glycérol, les N-mercaptoalkylamides de sucres tels que le N-(mercapto-2-éthyl)gluconamide, l'acide thiolactique et ses esters tels que le monothiolactate de glycérol, l'acide 3-mercaptopropionique et ses esters tels que le 3-mercaptopropionate de glycérol, l'acide thiomalique, l'acide 2-hydroxy 3-mercaptopropionique et ses esters, tels que le 2-hydroxy 3-mercaptopropionate de glycérol, la pantéthéine, le thioglycérol, les sulfites ou les bisulfites d'un métal alcalin ou alcalino-terreux, les N-(mercaptoalkyl)ω-hydroxyalkylamides décrits dans la demande de brevet EP-A-354835 et les N-mono- ou N,N-dialkylmercapto 4-butyramides décrits dans la demande de brevet EP-A-368763, les aminomercaptoalkylamides décrits dans la demande de brevet EP-A-432000, les dérivés des acides N-(mercaptoalkyl) succinamiques ou des N-(mercaptoalkyl) succiminides décrits dans la demande de brevet EP-A-465342, les alkylaminomercaptoalkylamides décrits dans la demande de brevet EP-A-514282, le mélange de thioglycolate d'hydroxy-2-propyle et de thioglycolate d'hydroxy-2-méthyl-1 éthyle décrit dans la demande de brevet FR-A-2679448, les N-mercaptoalkyl alcanediamides décrits dans la demande de brevet EP-A-653202.

On utilise de préférence l'acide thioglycolique, l'acide thiolactique, la cystéïne et ses dérivés, la cystéamine et ses dérivés, l'acide 3-mercaptopropionique, ainsi que leurs esters ou leurs sels, notamment le monothioglycolate de glycérol.

Ces agents actifs peuvent être utilisés seuls ou en mélange.

Lorsque l'on utilise l'acide thioglycolique, l'acide thiolactique, l'acide 3-mercaptopropionique, l'acide 2-hydroxy 3-mercaptopropionique, la cystéine ou la cystéamine, ou l'un de leurs sels ou de leurs dérivés, à titre d'agent réducteur, le pH de l'ensemble de la composition selon l'invention est de préférence compris entre 6 et 11,5 et encore plus préférentiellement entre 7 et 10.

Lorsque l'on utilise les esters de l'acide thioglycolique ou de l'acide thiolactique ou de l'acide 3-mercaptopropionique, de la cystéïne ou de l'acide 2-hydroxy 3-mercaptopropionique, à titre d'agent réducteur, le pH de l'ensemble de la composition selon l'invention est de préférence compris entre 5 et 10 et encore plus préférentiellement entre 6 et 9.

Les agents réducteurs mentionnés ci-avant sont généralement présents à une concentration qui peut aller de 1 à 20 % en poids par rapport au poids total de la compositon réductrice.

Les pH des compositions réductrices peuvent être ajustés classiquement par ajout d'agents basifiants en vue de rendre plus efficaces les agents réducteurs. Ces agents basifiants peuvent être choisis par exemple parmi la soude, l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires, ou un carbonate organique tel que le carbonate de guanidine, tous ces composés pouvant bien entendu être pris seuls ou en mélange.

L'antagoniste de substance P ou de CGRP permet en outre de réduire l'effet irritant de certains de ces agents basifiants.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique.

La composition réductrice peut également contenir un solvant tel que par exemple l'éthanol, le propanol, l'isopropanol ou encore le glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N,N'-diacétyl-cystamine, la cystine, la pantéthine, et les disulfures des N-(mercapto-alkyl) ω-hydroxyalkyl-amides décrits dans la demande de brevet EP-A-354835, les disulfures des N-mono ou N,N-dialkylmercapto-4 butyramides décrits dans la demande de brevet EP-A-368763, les disulfures des aminomercapto-alkylamides décrits dans la demande de brevet EP-A-432000, les disulfures des dérivés des acides N-(mercaptoalkyl)-succinamiques ou des N-(mercaptoalkyl)-succinimides décrits dans la demande de brevet EP-A-465342, les disulfures de alkylaminomercaptoalkylamides décrits dans la demande de brevet EP-A-514282 et les disulfures des N-mercaptoalkyl alcanediamides décrits dans la demande de brevet EP-A-653202. Ces disulfures sont généralement présents dans un rapport molaire de 0,5 à 2,5, et de préférence de 1 à 2, par rapport à l'agent réducteur.

Avant de procéder à l'étape suivante de rinçage (ii), il convient, de manière classique, de laisser reposer pendant quelques minutes, généralement entre 2 et 40 minutes, de préférence entre 5 et 30 minutes, les fibres kératiniques sur laquelle a été appliquée la composition réductrice, et ceci de façon à bien laisser le temps au réducteur d'agir correctement sur les fibres kératiniques. Cette phase d'attente est effectuée généralement en laissant reposer à l'air libre (température ambiante) les fibres kératiniques traitées, mais elle peut être également effectuée à une température plus élevée. Pendant cette phase d'attente, on prend soin que les fibres kératiniques ne sèchent pas complètement et restent humides jusqu'au moment de la mise en oeuvre de l'étape suivante.

Dans la deuxième étape du procédé (ii), les fibres kératiniques imprégnées de la composition réductrice sont donc rincées soigneusement par une composition aqueuse, éventuellement additionnée d'un antagoniste de substance P et/ou d'un antagoniste de CGRP.

Puis, dans une troisième étape (iii), on applique sur les fibres kératiniques ainsi rincées une composition oxydante dans le but de fixer la nouvelle forme imposée aux fibres kératiniques, cette composition oxydante pouvant contenir un antagoniste de substance P et/ou un antagoniste de CGRP.

On peut retirer des fibres kératiniques les moyens mécaniques (rouleaux, bigoudis et analogues) qui maintenaient sous tension et dans la forme désirée les fibres kératiniques tout au long du traitement avant ou après l'étape de fixation.

La composition oxydante contient un agent oxydant qui peut être choisi parmi l'eau oxygénée, un bromate alcalin, un persel, un chlorite ou un polythionate ou leur mélange, tel qu'un mélange de bromate alcalin et d'un persel. On peut utiliser par exemple le bromate de potassium, le perborate de sodium, le chlorite de sodium.

La concentration en eau oxygénée peut varier de 1 à 10 volumes, mais est de préférence de 8 volumes ; la concentration en bromate alcalin est généralement de 1 à 12 % et celle en persel de 0,1 à 15 % en poids par rapport au poids total de la composition oxydante.

Le pH de la composition oxydante est généralement compris entre 2 et 10.

La composition oxydante peut contenir des additifs cosmétiques bien connus pour ce type de composition.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions réductrice et oxydante utilisées selon l'invention est de préférence l'eau ou une solution hydroalcoolique d'un alcool inférieur tel que l'éthanol, l'isopropanol ou le butanol.

L'eau oxygénée peut être stabilisée par exemple par la phénacétine, l'acétanilide, les phosphates mono et trisodiques ou par le sulfate d'hydroxy-8 quinoléine.

La composition oxydante peut également contenir des agents alcalinisants ou acidifiants, des agents conservateurs, des agents séquestrants, des opacifiants.

Enfin, les fibres kératiniques imprégnées de la composition oxydante sont rincées soigneusement, généralement à l'eau éventuellement additionnée d'un antagoniste de substance P et/ou d'un antagoniste de CGRP.

On obtient finalement une chevelure présentant la mise en forme souhaitée, sans qu'apparaisse irritation ou inconfort.

Les quantités des différents constituants des compositions selon l'invention sont celles classiquement utilisées dans les domaines considérés.

Les exemples qui suivent sont destinés à illustrer l'invention sans pour autant en limiter la portée.

### Exemple 1 : Composition pour permanente

### A. Composition réductrice

| | |
|---|---|
| - acide thioglycolique | 9,2 g |
| - carbonate de sodium | 1 g |
| - monoéthanolamine qs | pH 8,5 |
| - mélange cocoylamidopropylbetaïne/monolaurate de glycérol (25/5) vendu sous la dénomination "TEGOBETAÏNE HS" par la société Goldschmidt à 30% de matière active | 0,3 g MA |
| - Chlorure de strontium | 5 g |
| - eau déminéralisée qsp | 100 g |

### B. Composition oxydante

| | |
|---|---|
| - eau oxygénée qsp | 8 volumes |
| - acide citrique qs | pH 3 |
| - eau déminéralisée qsp | 100 g |

Le mode opératoire d'utilisation est suivant : on applique sur les cheveux enroulés et humides (diamètre des rouleaux : 9 mm) la composition réductrice, puis on pose un bonnet plastique sur la chevelure et on attend 15 minutes. Puis on enlève le bonnet et l'on rince les cheveux. Ensuite, on applique sur les cheveux la composition oxydante ; on laisse agir pendant 10 minutes, puis on retire les rouleaux. Puis, on rince à l'eau et enfin on sèche les cheveux.

### Exemple 2 :

On réalise la même composition réductrice que dans l'exemple 1, en remplaçant les 5 g de chlorure de strontium par 0,05 g de Spantide II. La composition oxydante est la même que dans l'exemple 1.

### Exemple 3:

On réalise la même composition réductrice que dans l'exemple 1, en remplaçant les 5 g de chlorure de strontium par 0,5 g de CGRP 8-37. La composition oxydante est la même que dans l'exemple 1.

### Exemple 4 :

On réalise la même composition réductrice que dans l'exemple 1, et une composition oxydante comprenant en plus 5 g d'un extrait d'*Iris pallida* préparé de la manière suivante :

Des cellules indifférenciées d'*Iris pallida* cultivées *in vitro* en conditions axéniques sont récupérées après culture en Erlenmeyer ou en fermenteur par filtration sur tamis de 50 µm. A 55 g de matière fraîche ainsi obtenue, on ajoute 27,5 ml d'eau déminéralisée. L'ensemble est broyé au Turax à 24000 T/min durant 1 minute à 4°C (bain de glace). Le broyat est centrifugé à 4°C. Le surnageant est filtré à 0,22 µm (filtration stérilisante). L'extrait ainsi préparé est conservé à 4°C. Il renferme environ 15 g de matière sèche par litre.

### Exemple 5 : Composition pour permanente

### Composition contenant l'antagoniste de substance P (A)

- Lotion hydroalcoolique (eau/éthanol: 90/10) à 6,3 % de chlorure de strontium.

### Composition réductrice (B)

| | |
|---|---|
| - acide thioglycolique | 9,4 g |
| - Ammoniaque à 20 % en ammoniac | 11,5 g |
| - Bicarbonate d'ammonium | 5,8 g |
| - Sel pentasodique d'EDTA à 40 % en solution aqueuse | 0,2 g |
| - Cocoyl bétaïne | 0,45 g |
| - Laurate de polyéthylèneglycol 6 OE (Laurynol 13 vendu par la société Interchimie) | 0,09 g |
| - Polycondensat tétraméthyl hexaméthylènediamine/dichloro-1,3-propylène en solution aqueuse à 60 % | 2,0 g |
| - Dithioglycolate de diammonium en solution aqueuse à 48 % | 7,5 g |
| - Alcool oléique oxyéthyléné 10 OE | 1,5 g |
| - Eau déminéralisée qsp | 100 g |

### Composition oxydante (C)

| | |
|---|---|
| - Eau oxygénée à 200 volumes | 4,8 g |
| - Stabilisants qs | |
| - Agent acidifiant qsp | pH 3 |
| - Eau déminéralisée qsp | 100 g |

Pour effectuer la permanente, on a d'abord appliqué la composition (A) contenant l'antagoniste de substance P, puis on a suivi le mode opératoire décrit dans les exemples précédents.

Un test a été réalisé pour mettre en évidence contre le placebo (A') l'effet apaisant de la composition (A) appliquée avant la permanente. Le test a été réalisé sur 13 sujets.

Le placebo (A') consistait en une solution hydroalcoolique (eau/éthanol : 90/10).

Le test a consisté à appliquer, avant la permanente, sur une demi-tête la lotion (A) et sur l'autre demi-tête la lotion (A') et à évaluer les sensations d'inconfort (picotements, démangeaisons, brûlures) avant et pendant les étapes de la permanente.

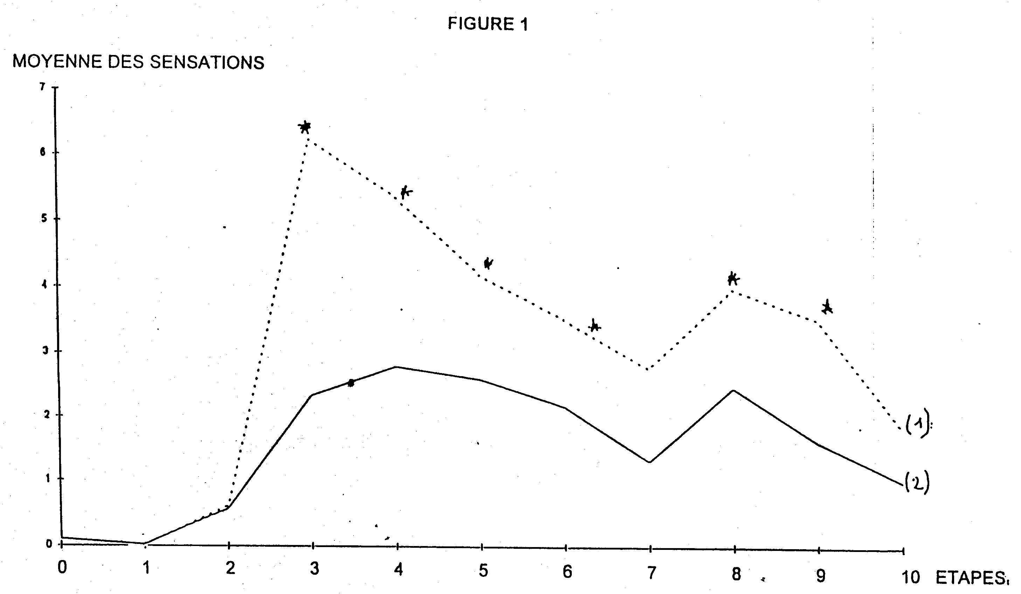

Les résultats du test sont présentés sur la figure 1 qui donne en abscisse les étapes (1 à 10) de la permanente, en fonction de la moyenne des sensations d'inconfort, en ordonnée, ces sensations étant évaluées de 0 à 7 (pas de sensations à 0 et beaucoup d'inconfort à 7). La courbe (1) en pointillés est celle obtenue avec le placebo et la courbe (2) en traits pleins est celle obtenue avec la lotion selon l'invention.

Les étapes ont été les suivantes :
1. Application par demi-tête de la lotion (A) ou (A') ;
2. Enroulement des cheveux sur des rouleaux ;
3. Application de la composition (B) ;
4. 2 minutes après application de la composition (B) ;
5. 5 minutes après application de la composition (B) ;
6. 10 minutes après application de la composition (B) ;
7. Rinçage ;
8. Application de la composition (C) ;
9. 5 minutes après application de la composition (C) ;
10. Rinçage.

La figure 1 met en évidence une nette diminution des sensations d'inconfort quand le traitement des cheveux est précédé de l'application de la composition (A) selon l'invention, contenant un antagoniste de substance P.

### Exemple 6 : Test à la monoéthanolamine

On a réalisé un test mettant en évidence l'effet apaisant d'un antagoniste de substance P après induction d'une réaction par application de monoéthanolamine sur 14 sujets ayant le cuir chevelu sensible.

Le test a consisté à traiter préalablement le cuir chevelu par demi-tête soit par une lotion hydroalcoolique (eau/éthanol : 90/10) à 5 % de chlorure de strontium soit par un placebo constitué d'une solution hydroalcoolique (eau/éthanol : 90/10), à appliquer ensuite une solution aqueuse à 10 % de monoéthanolamine et à évaluer cliniquement les sensations d'inconfort (picotements, démangeaisons, brûlures) jusqu'à 15 minutes après l'application de la solution aqueuse de monoéthanolamine.

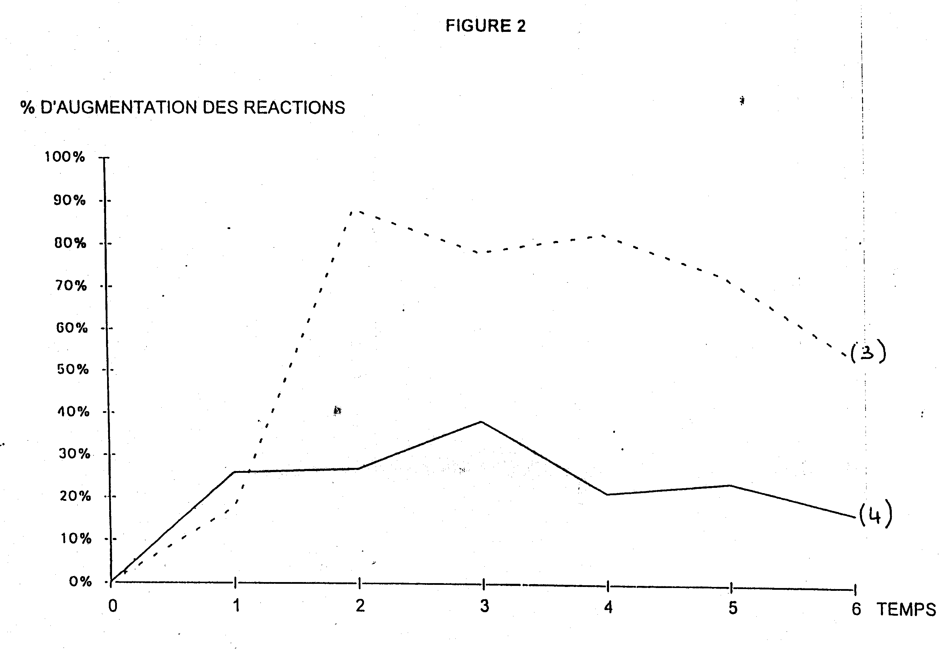

Les résultats du test sont présentés sur la figure 2 qui donne en abscisse le temps (exprimé de 0 à 6), en fonction de la moyenne du pourcentage d'augmentation des réactions en ordonnée. La courbe (3) en pointillés est celle obtenue avec le placebo et la courbe (4) en traits pleins est celle obtenue avec la lotion selon l'invention.

Le temps en abscisse correspond aux temps réels suivants :
1. Après application de la lotion selon l'invention ou du placebo et avant application de la solution de monoéthanolamine ;
2. 30 secondes après application de la solution de monoéthanolamine ;
3. 2 minutes après application de la solution de monoéthanolamine ;
4. 5 minutes après application de la solution de monoéthanolamine ;
5. 10 minutes après application de la solution de monoéthanolamine ;
6. 15 minutes après application de la solution de monoéthanolamine ;

La figure 2 met en évidence une nette diminution des réactions quand le traitement des cheveux est précédé de l'application de la lotion selon l'invention, contenant un antagoniste de substance P.

## Revendications

1. Composition notamment cosmétique contenant dans un milieu physiologiquement acceptable au moins un antagoniste de substance P et au moins un actif à effet secondaire irritant, caractérisée en ce que l'actif à effet secondaire irritant est un agent réducteur et/ou un agent oxydant à l'exception des peroxydes et des crésols, à condition que l'actif soit différent d'un thiol quand l'antagoniste de substance P est un dérivé d'éthylène diamine.

2. Composition selon la revendication 1, caractérisée en ce qu'elle comprend en outre au moins un antagoniste de CGRP.

3. Composition selon la revendication 1, caractérisée en ce que l'agent réducteur est choisi parmi les sulfites, les bisulfites, les alkyl-phosphines, les thiols et leurs mélanges.

4. Composition notamment cosmétique contenant dans un milieu physiologiquement acceptable au moins un antagoniste de CGRP et au moins un actif à effet secondaire irritant, caractérisée en ce que l'actif à effet secondaire irritant est un agent réducteur et/ou un agent oxydant à l'exception des peroxydes, des crésols et des thiols.

5. Composition selon la revendication 4, caractérisée en ce que l'agent réducteur est choisi parmi les sulfites, les bisulfites, les alkyl-phosphines et leurs mélanges.

6. Composition selon la revendication 4 ou 5, caractérisée en ce qu'elle comprend en outre au moins un antagoniste de substance P.

7. Composition selon l'une quelconque des revendications précédentes, caractérisée en ce qu'elle constitue une composition pour la déformation permanente des fibres kératiniques.

8. Composition selon l'une quelconque des revendications précédentes, caractérisée en ce que l'antagoniste de substance P est choisi parmi les peptides, les composés comprenant au moins un hétérocycle, les composés azotés comprenant un ou plusieurs cycles benzèniques, les sels de métaux monovalents, divalents ou trivalents, les extraits d'origine végétale, les extraits d'origine bactérienne et leurs mélanges.

9. Composition selon la revendication 8, caractérisée en ce que le peptide est le sendide ou le spantide II.

10. Composition selon la revendication 8, caractérisée en ce que le composé comprenant au moins un hétérocycle est un composé hétérocyclique azoté choisi parmi les dérivés de 2-tricyclyl-2-amino-éthane, les dérivé de spirolactame, les dérivés de quinuclidine, les dérivés azacycliques, les dérivés d'aminopyrrolidine, les dérivés de pipéridine, les aminoazahétérocycles, les dérivés d'isoindole et leurs mélanges.

11. Composition selon la revendication 8, caractérisée en ce que le composé comprenant au moins un hétérocycle est un composé hétérocyclique oxygéné ou soufré choisi parmi les dérivés du furanne, les dérivés du benzofuranne, les dérivés du thiophène, les dérivés du benzothiophène, et notamment les tétrazolyl-benzofuranne-carboxamides ou les tétrazolyl-benzothiophène-carboxamides, et leurs mélanges.

12. Composition selon la revendication 8, caractérisée en ce que le composé azoté comprenant au moins un cycle benzénique est un dérivé d'éthylène diamine.

13. Composition selon la revendication 8, caractérisée en ce que le sel de métal monovalent, divalent ou trivalent, est choisi parmi les chlorures, carbonates, bicarbonates, borates, nitrates, acétates, hydroxydes, sulfates, persulfates, glycérophosphates, les sels d'α-hydroxyacides, les sels d'acides de fruits, les sels d'acides aminés et les sels d'acides gras de cobalt, de béryllium, de magnésium, de strontium, de calcium, de baryum, de lanthanides notamment de lanthane et de gadolinium, d'yttrium, de zinc, de manganèse, de cuivre, de rubidium et/ou de lithium, et leurs mélanges.

14. Composition selon la revendication 13, caractérisée en ce que le sel est un sel de strontium.

15. Composition selon la revendication 8, caractérisée en ce que l'extrait d'origine végétale est un extrait d'iridacée.

16. Composition selon l'une quelconque des revendications 2 à 15, caractérisée en ce que l'antagoniste de CGRP est choisi parmi le CGRP 8-37, les anticorps anti-CGRP, et leurs mélanges.

17. Composition selon l'une quelconque des revendications précédentes, caractérisée en ce que l'antagoniste est utilisé en une quantité allant de 0,000001 à 30 % en poids par rapport au poids total de la composition.

18. Composition selon l'une quelconque des revendications précédentes, caractérisée en ce que l'antagoniste est utilisé en une quantité allant de 0,0001 à 10 % en poids par rapport au poids total de la composition.

19. Composition selon l'une quelconque des revendications précédentes, caractérisée par le fait que l'agent oxydant est choisi parmi les bromates alcalins, les persels, les chlorites, les polythionates et leurs mélanges.

20. Composition selon l'une quelconque des revendications précédentes, caractérisée par le fait que l'agent oxydant est choisi parmi le perborate de sodium, le bromate de potassium, le chlorite de sodium et leurs mélanges.

21. Composition selon l'une quelconque des revendications précédentes, caractérisée par le fait que le milieu est un milieu aqueux constitué par de l'eau ou un mélange d'eau et d'un solvant organique.

22. Composition selon l'une quelconque des revendications précédentes, caractérisée par le fait que la composition renferme en outre au moins un adjuvant choisi parmi les agents alcalinisants ou acidifiants, les agents conservateurs, les agents séquestrants, les opacifiants.

23. Procédé de traitement des fibres kératiniques, en particulier des cheveux, en vue d'obtenir une déformation permanente de ces fibres, caractérisé par le fait qu'il comprend les étapes suivantes :
i) application sur les fibres kératiniques d'une composition réductrice, les moyens nécessaires à la mise sous tension mécanique des fibres kératiniques étant mis en oeuvre avant, pendant ou après cette application de la composition réductrice,
ii) après action de la composition réductrice, rinçage des fibres kératiniques,
iii) application sur les fibres kératiniques d'une composition oxydante,
iv) séparation des moyens de mise sous tension avant ou après l'étape iii,
v) rinçage éventuel des fibres kératiniques,
vi) application sur les fibres kératiniques d'une composition contenant au moins un antagoniste choisi parmi les antagonistes de substance P et les antagonistes de CGRP, au cours de l'une au moins des étapes i à v et/ou après l'une au moins de ces étapes i à v.

24. Kit pour le traitement des fibres kératiniques, en particulier des cheveux, en vue d'obtenir une déformation permanente de ces fibres, comprenant une première composition contenant un agent réducteur et une seconde composition contenant un agent oxydant, les deux compositions, destinées à être appliquées l'une après l'autre sur les fibres kératiniques, étant conditionnées séparément, l'une au moins de ces compositions contenant un antagoniste choisi parmi les antagonistes de substance P et les antagonistes de CGRP.

25. Kit pour le traitement des fibres kératiniques, en particulier des cheveux, en vue d'obtenir une déformation permanente de ces fibres, comprenant une première composition contenant un agent réducteur, une seconde composition contenant un agent oxydant, une troisième composition contenant un antagoniste choisi parmi les antagonistes de substance P et les antagonistes de CGRP, les trois compositions, destinées à être appliquées les unes après les autres sur les fibres kératiniques, étant conditionnées séparément.

## Claims

1. Composition, in particular cosmetic composition, containing, in a physiologically acceptable medium, at least one substance P antagonist and at least one active agent with an irritant side effect, characterized in that the active agent with an irritant side effect is a reducing agent and/or an oxidizing agent, with the exception of peroxides and cresols, provided that the active agent is other than a thiol when the substance P antagonist is an ethylene diamine derivative.

2. Composition according to Claim 1, characterized in that it additionally comprises at least one CGRP antagonist.

3. Composition according to Claim 1, characterized in that the reducing agent is chosen from sulphites, bisulphites, alkylphosphines, thiols and their mixtures.

4. Composition, in particular cosmetic composition, containing, in a physiologically acceptable medium, at least one CGRP antagonist and at least one active agent with an irritant side effect, characterized in that the active agent with an irritant side effect is a reducing agent and/or an oxidizing agent, with the exception of peroxides, cresols and thiols.

5. Composition according to Claim 4, characterized in that the reducing agent is chosen from sulphites, bisulphites, alkylphosphines and their mixtures.

6. Composition according to Claim 4 or 5, characterized in that it additionally comprises at least one substance P antagonist.

7. Composition according to any one of the preceding claims, characterized in that it constitutes a composition for the permanent deformation of keratinous fibres.

8. Composition according to any one of the preceding claims, characterized in that the substance P antagonist is chosen from peptides, compounds comprising at least one heterocycle, nitrogen-containing compounds comprising one or a number of benzene rings, salts of monovalent, divalent or trivalent metals, extracts of plant origin, extracts of bacterial origin and their mixtures.

9. Composition according to Claim 8, characterized in that the peptide is sendide or spantide II.

10. Composition according to Claim 8, characterized in that the compound comprising at least one heterocycle is a nitrogen-containing heterocyclic compound chosen from 2-tricyclyl-2-aminoethane derivatives, spirolactam derivatives, quinuclidine derivatives, azacyclic derivatives, aminopyrrolidine derivatives, piperidine derivatives, aminoazaheterocycles, isoindole derivatives and their mixtures.

11. Composition according to Claim 8, characterized in that the compound comprising at least one heterocycle is an oxygen-containing or sulphur-containing heterocyclic compound chosen from furan derivatives, benzofuran derivatives, thiophene derivatives, benzothiophene derivatives, and in particular tetrazolylbenzofurancarboxamides or tetrazolylbenzothiophenecarboxamides, and their mixtures.

12. Composition according to Claim 8, characterized in that the nitrogen-containing compound comprising at least one benzene ring is an ethylenediamine derivative.

13. Composition according to Claim 8, characterized in that the monovalent, divalent or trivalent metal salt is chosen from cobalt, beryllium, magnesium, strontium, calcium, barium, lanthanide, in particular lanthanum and gadolinium, yttrium, zinc, manganese, copper, rubidium and/or lithium chlorides, carbonates, bicarbonates, borates, nitrates, acetates, hydroxides, sulphates, persulphates, glycerophosphates, salts of α-hydroxy acids, salts of fruit acids, salts of amino acids and salts of fatty acids, and their mixtures.

14. Composition according to Claim 13, characterized in that the salt is a strontium salt.

15. Composition according to Claim 8, characterized in that the extract of plant origin is an Iridacea extract.

16. Composition according to any one of Claims 2 to 15, characterized in that the CGRP antagonist is chosen from CGRP 8-37, anti-CGRP antibodies and their mixtures.

17. Composition according to any one of the preceding claims, characterized in that the antagonist is used in an amount ranging from 0.000001 to 30% by weight with respect to the total weight of the composition.

18. Composition according to any one of the preceding claims, characterized in that the antagonist is used in an amount ranging from 0.0001 to 10% by weight with respect to the total weight of the composition.

19. Composition according to any one of the preceding claims, characterized in that the oxidizing agent is chosen from alkali metal bromates, from persalts, from chlorites, from polythionates and their mixtures.

20. Composition according to any one of the preceding claims, characterized in that the oxidizing agent is chosen from sodium perborate, potassium bromate, sodium chlorite and their mixtures.

21. Composition according to any one of the preceding claims, characterized in that the medium is an aqueous medium composed of water or a mixture of water and of an organic solvent.

22. Composition according to any one of the preceding claims, characterized in that the composition additionally contains at least one adjuvant chosen from basifying or acidifying agents, preserving agents, sequestering agents or opacifiers.

23. Process for the treatment of keratinous fibres, in particular hair, for the purpose of obtaining a permanent deformation of these fibres, characterized in that it comprises the following stages:
i) application to the keratinous fibres of a reducing composition, the means necessary for placing the keratinous fibres under mechanical tension being applied before, during or after this application of the reducing composition,
ii) once the reducing composition has taken effect, rinsing the keratinous fibres,
iii) application to the keratinous fibres of an oxidizing composition,
iv) removal of the means for placing under tension before or after stage iii,
v) optional rinsing of the keratinous fibres,
vi) application to the keratinous fibres of a composition containing at least one antagonist chosen from substance P antagonists and CGRP antagonists, during at least one of stages i to v and/or after at least one of these stages i to v.

24. Kit for the treatment of keratinous fibres, in particular of hair, for the purpose of obtaining a permanent deformation of these fibres, comprising a first composition containing a reducing agent and a second composition containing an oxidizing agent, the two compositions, intended to be applied one after the other to the keratinous fibres, being packaged separately, at least one of these compositions containing an antagonist chosen from substance P antagonists and CGRP antagonists.

25. Kit for the treatment of keratinous fibres, in particular of hair, for the purpose of obtaining a permanent deformation of these fibres, comprising a first composition containing a reducing agent, a second composition containing an oxidizing agent and a third composition containing an antagonist chosen from substance P antagonists and CGRP antagonists, the three compositions, intended to be applied one after the other to the keratinous fibres, being packaged separately.

## Patentansprüche

1. Zusammensetzung, insbesondere kosmetische Zusammensetzung, die in einem physiologisch akzeptablen Medium mindestens einen Antagonisten der Substanz P und mindestens einen Wirkstoff mit reizender Nebenwirkung enthält, die dadurch gekennzeichnet ist, daß der Wirkstoff mit reizender Nebenwirkung ein Reduktionsmittel und/oder ein Oxidationsmittel mit Ausnahme von Peroxiden und Kresolen ist mit der Maßgabe, daß der Wirkstoff verschieden von Thiolen ist, wenn der Antagonist der Substanz P ein Ethylendiamin-Derivat ist.

2. Zusammensetzung nach Anspruch 1, dadurch gekennzeichnet, daß sie außerdem mindestens einen Antagonisten von CGRP enthält.

3. Zusammensetzung nach Anspruch 1, dadurch gekennzeichnet, daß das Reduktionsmittel unter Sulfiten, Hydrogensulfiten, Alkylphosphinen, Thiolen und deren Gemischen ausgewählt ist.

4. Zusammensetzung, insbesondere kosmetische Zusammensetzung, die in einem physiologisch akzeptablen Medium mindestens einen Antagonisten von CGRP und mindestens einen Wirkstoff mit reizender Nebenwirkung enthält, dadurch gekennzeichnet, daß der Wirkstoff mit reizender Nebenwirkung ein Reduktionsmittel und/oder ein Oxidationsmittel mit Ausnahme von Peroxiden, Kresolen und Thiolen ist.

5. Zusammensetzung nach Anspruch 4, dadurch gekennzeichnet, daß das Reduktionsmittel unter Sulfiten, Hydrogensulfiten, Alkylphosphinen und deren Gemischen ausgewählt ist.

6. Zusammensetzung nach Anspruch 4 oder 5, dadurch gekennzeichnet, daß sie außerdem mindestens einen Antagonisten der Substanz P enthält.

7. Zusammensetzung nach einem der vorhergehenden Ansprüche, dadurch gekennzeichnet, daß sie eine Zusammensetzung für die Dauerwellverformung von Keratinfasern ist.

8. Zusammensetzung nach einem der vorhergehenden Ansprüche, dadurch gekennzeichnet, daß der Antagonist der Substanz P unter Peptiden, Verbindungen, die mindestens einen Heterocyclus enthalten, stickstoffhaltigen Verbindungen, die einen oder mehrere Benzolringe enthalten, Salzen einwertiger, zweiwertiger oder dreiwertiger Metalle, Extrakten pflanzlicher Herkunft, Extrakten bakterieller Herkunft und deren Gemischen ausgewählt ist.

9. Zusammensetzung nach Anspruch 8, dadurch gekennzeichnet, daß es sich bei dem Peptid um Sendide oder Spantide II handelt.

10. Zusammensetzung nach Anspruch 8, dadurch gekennzeichnet, daß die Verbindung, die mindestens einen Heterocyclus enthält, eine stickstoffhaltige heterocyclische Verbindung ist, die unter 2-Tricyclyl-2-aminoethan-Derivaten, Spirolactam-Derivaten, Chinuclidin-Derivaten, azacyclischen Derivaten, Aminopyrrolidin-Derivaten, Piperidin-Derivaten, Aminoazaheterocyclen, Isoindol-Derivaten und deren Gemischen ausgewählt ist.

11. Zusammensetzung nach Anspruch 8, dadurch gekennzeichnet, daß die Verbindung, die mindestens einen Heterocyclus enthält, eine sauerstoffhaltige oder schwefelhaltige heterocyclische Verbindung ist, die unter Furan-Derivaten, Benzofuran-Derivaten, Thiophen-Derivaten, Benzothiophen-Derivaten und insbesondere den Tetrazolylbenzofurancarboxamiden und Tetrazolylbenzothiophencarboxamiden und deren Gemischen ausgewählt ist.

12. Zusammensetzung nach Anspruch 8, dadurch gekennzeichnet, daß die stickstoffhaltige Verbindung, die mindestens einen Benzolring enthält, ein Ethylendiamin-Derivat ist.

13. Zusammensetzung nach Anspruch 8, dadurch gekennzeichnet, daß das Salz eines einwertigen, zweiwertigen oder dreiwertigen Metalls unter den Chloriden, Carbonaten, Hydrogencarbonaten, Boraten, Nitraten, Acetaten, Hydroxiden, Sulfaten, Persulfaten, Glycerinphosphaten, den Salzen von α-Hydroxysäuren, den Salzen von Fruchtsäuren, den Salzen von Aminosäuren und den Salzen von Fettsäuren von Cobalt, Beryllium, Magnesium, Strontium, Calcium, Barium, von Lanthaniden, insbesondere Lanthan und Gadolinium, Yttrium, Zink, Mangan, Kupfer, Rubidium und/oder Lithium und deren Gemischen ausgewählt ist.

14. Zusammensetzung nach Anspruch 13, dadurch gekennzeichnet, daß das Salz ein Strontiumsalz ist.

15. Zusammensetzung nach Anspruch 8, dadurch gekennzeichnet, daß der Extrakt pflanzlicher Herkunft ein Iridacea-Extrakt ist.

16. Zusammensetzung nach einem der Ansprüche 2 bis 15, dadurch gekennzeichnet, daß der Antagonist von CGRP unter CGRP 8-37, Anti-CGRP-Antikörpern und deren Gemischen ausgewählt ist.

17. Zusammensetzung nach einem der vorhergehenden Ansprüche, dadurch gekennzeichnet, daß der Antagonist in einer Menge von 0,000001 bis 30 Gew.-%, bezogen auf das Gesamtgewicht der Zusammensetzung, verwendet wird.

18. Zusammensetzung nach einem der vorhergehenden Ansprüche, dadurch gekennzeichnet, daß der Antagonist in einer Menge von 0,0001 bis 10 Gew.-%, bezogen auf das Gesamtgewicht der Zusammensetzung, verwendet wird.

19. Zusammensetzung nach einem der vorhergehenden Ansprüche, dadurch gekennzeichnet, daß das Oxidationsmittel unter Alkalibromaten, Persalzen, Chloriten, Polythionaten und deren Gemischen ausgewählt ist.

20. Zusammensetzung nach einem der vorhergehenden Ansprüche, dadurch gekennzeichnet, daß das Oxidationsmittel unter Natriumperborat, Kaliumbromat, Natriumchlorit und deren Gemischen ausgewählt ist.

21. Zusammensetzung nach einem der vorhergehenden Ansprüche, dadurch gekennzeichnet, daß das Medium ein wäßriges Medium ist, das aus Wasser oder einem Gemisch von Wasser und einem organischen Lösungsmittel besteht.

22. Zusammensetzung nach einem der vorhergehenden Ansprüche, dadurch gekennzeichnet, daß die Zusammensetzung außerdem mindestens einen Hilfsstoff enthält, der unter Alkalisierungsmitteln oder Säuerungsmitteln, Konservierungsmitteln, Maskierungsmitteln, Trübungsmitteln ausgewählt ist.

23. Verfahren zur Behandlung von Keratinfasern, insbesondere der Haare, zur Durchführung einer Dauerwellverformung dieser Fasern, dadurch gekennzeichnet, daß es die folgenden Schritte umfaßt:
i) Auftragen einer reduzierenden Zusammensetzung auf die Keratinfasern, wobei die Mittel, die erforderlich sind, um die Keratinfasern unter mechanische Spannung zu setzen, vor, während oder nach Auftragen der reduzierenden Zusammensetzung angewendet werden,
ii) Spülen der Keratinfasern nach Einwirkung der reduzierenden Zusammensetzung,
iii) Auftragen einer oxidierenden Zusammensetzung auf die Keratinfasern,
iv) Entfernen der Mittel, mit denen die Keratinfasern unter Spannung gesetzt wurden, vor oder nach Schritt iii),
v) gegebenenfalls Spülen der Keratinfasern,
vi) Auftragen einer Zusammensetzung, die mindestens einen Antagonisten enthält, der unter den Antagonisten der Substanz P und den Antagonisten von CGRP ausgewählt ist, während mindestens einem der Schritte i bis v und/oder nach mindestens einem dieser Schritte i bis v auf die Keratinfasern.

24. Kit zur Behandlung von Keratinfasern, insbesondere der Haare, zur Erzielung einer Dauerwellverformung dieser Fasern, der eine erste Zusammensetzung, die ein Reduktionsmittel enthält, und eine zweite Zusammensetzung, die ein Oxidationsmittel enthält, umfaßt, wobei die beiden Zusammensetzungen, die dafür vorgesehen sind, nacheinander auf die Keratinfasern aufgetragen zu werden, getrennt voneinander konfektioniert sind, wobei mindestens eine dieser Zusammensetzungen einen Antagonisten enthält, der unter den Antagonisten der Substanz P und den Antagonisten von CGRP ausgewählt ist.

25. Kit zur Behandlung von Keratinfasern, insbesondere der Haare, zur Durchführung einer Dauerwellverformung dieser Fasern, der eine erste Zusammensetzung, die ein Reduktionsmittel enthält, eine zweite Zusammensetzung, die ein Oxidationsmittel enthält, eine dritte Zusammensetzung, die einen Antagonisten enthält, der unter den Antagonisten der Substanz P und den Antagonisten von CGRP ausgewählt ist, umfaßt, wobei die drei Zusammensetzungen, die dafür vorgesehen sind, nacheinander auf die Keratinfasern aufgetragen zu werden, getrennt voneinander konfektioniert sind.
